# EUROPEAN PATENT SPECIFICATION

(11) **EP 0 426 358 B1**
(45) Date of publication and mention of the grant of the patent: **12.05.1999**
(21) Application number: 90311596.2
(22) Date of filing: 23.10.1990
(51) Int. Cl.: A61B 5/00

(54) **A non-invasive method and apparatus for measuring blood chemical concentration**
Verfahren und Gerät zur nichtinvasiven Messung des Gehaltes eines chemischen Stoffes im Blut
Méthode et appareil de mesure non invasive de la concentration d'une substance chimique dans le sang

(30) Priority: 28.10.1989 KR 8915584; 24.07.1990 KR 9011241
(43) Date of publication of application: 08.05.1991
(73) Proprietor: Yang, Won Suck, Seoul (KR); Kim, Yoon Ok, Seoul (KR)
(72) Inventor: Yang, Won Suck, Seoul (KR); Kim, Yoon Ok, Seoul (KR)
(74) Representative: Rackham, Stephen Neil

(56) References cited:
- EP-A- 74 428
- EP-A- 0 160 768
- EP-A- 0 274 403
- EP-A- 0 317 121
- DE-A- 3 328 862
- APPLIED SPECTROSCOPY. vol. 43, no. 5, July 1989, BALTIMORE US pages 834 - 839; MAY: "Computer processing of tunable diode laser spectra."

## Description

The present invention relates to a method and apparatus for measuring blood chemical concentration, and more particularly, to a non-invasive technique for measuring eg blood glucose concentration using near-infrared radiation diffuse-reflection laser spectroscopy.

Generally, diabetics measure blood glucose concentration two to eight times daily using a portable measurement apparatus consisting of an injector (to obtain a blood sample) and test paper (to measure the amount of glucose in the blood). This known as the "enzymatic" method or test.

The enzymatic test for glucose concentration is undesirable both because it requires the blood be drawn and because it is expensive. Less expensive techniques based on test paper have been introduced, but they are less accurate and still require that blood be drawn. Accordingly, research has been conducted to address these problems.

It is desired to provide a method for measuring look glucose concentration that is accurate and does not require the drawing of blood.

EP-A-160768 discloses a spectroscopic method and apparatus for non-invasive measurement of blood glucose in tissues. Radiation beams of two different selected bands of the near infra-red are directed into the subject and the transmitted light, which is partly absorbed by the glucose molecules, is collected using an integrating halfsphere. One of the radiation beams is used for the primary measurement, the other one for a reference measurement.

In a variation of that apparatus, the reference beam is substituted by a reference measurement in a depth, different to the depth used for the primary measurement. In this case, the diffusely reflected light collected by the halfsphere is used for the glucose determination. The different depths are obtained by different angles of the readiation beams, directed into the tissue. The device uses a moveable mirror to produce the different angles necessary for the glucose determination.

In both cases, reference measurements are needed, which can only be otained rather by voluminous devices, either by using two radiation beams of different wavelength or by using a moveable mirror with a motor.

A major aspect of the present invention is to provide a portable and easy-to-use apparatus. This, however, is not obtainable while reference measurements as described in EP-A-160768 are necessary. Therefore, it is one aspect of the present invention, to provide an apparatus that does not need a reference measurement, motors, moveable mirrors or other voluminous parts of the that kind. Utilising overtone vibrations of the to-be-anlysed molcules in the blood may be helpful for analyses which do not need reference measurements and therefore, the device can be very small i.e. portable.

EP-A-317121 discloses a spectroscopic apparatus for non-invasive measurement of, e.g., blood sugar concentration. Infra-red radiation in selected bands from 950nm to 1,450nm is directed into the subject under measurement, and the reflected light collected using an integrating sphere. Measured absorption levels at different wavelengths and intensities are compared with stored values to determine blood sugar concentration levels.

Preferred aspects of the present invention provide a non-invasive technique for measuring blood glucose concentration to eliminate the need for drawing blood for this measurement. Other aspects, of the present invention provide a convenient, inexpensive, portable, easy-to-use apparatus for measuring blood glucose concentration.

### SUMMARY OF THE INVENTION

According to a first aspect of the present invention, there is provided a method for measuring the concentration of a chemical carried in the blood stream including the steps of:
irradiating a blood vessel carrying the blood stream with near-infrared light having a wavelength of between substantially 1.3µm and substantially 1.9µm;
detecting radiation dispersed or diffusedly rejected by the blood using a photodetector; and,
analysing the detected radiation using diffuse-reflection spectroscopy responsive to vibrational, rotational and translational motion of the molecules of the said chemical utilising overtone vibrations and a combination of other types of vibration to determine the concentration of the chemical in the blood, the method characterised in that:
the step of irradiating is carried out with light from a laser diode;
the step of analysing the detected radiation is carried out by comparison of a detected value with a stored calibration curve; and,
the apparatus used to measure the concentration is a portable apparatus.

According to a second aspect of the present invention, there is provided an apparatus for measuring non-invasively blood glucose concentratio, the apparatus comprising:
means to irradiate a blood vessel carrying the blood stream with light at a wavelength of between substantially 1.3µm and substantially 1.9µm;
an integrating unit which integrates light dispersed and diffusedly reflected from the blood;
a detector which amplifies a signal by means of a preamplifier after converting photons integrated by said integrating element into an electrical analog measurement value;
an A/D converter for converting said electrical analog measurement value into a digital measurement value; and
processing means including means for calculating and computing blood glucose concentration;
   characterised in that:
the apparatus has a width x length x height less than 170 mm x 80 mm x 25 mm;
the apparatus is arranged to irradiate the blood with light from a laser diode;
the processing means are adapted for controlling the flow of current supplied from a laser diode power supply (4) to the laser diode; and,
the means for calculating and computing are adapted for comparing an electrical analog measurement value detected and converted by the detector with a calibration curve stored in memory of said processing means,
and in that the apparatus further comprises:
a D/A converter for controlling the laser diode power supply by converting a digital control signal to an analog control signal, said laser diode consisting of a plurality of diodes for emitting light of different wavelengths or emitting light of the same wavelength in accordance with the current supplied from said laser diode power supply, the processing means and A/D converter being arranged in combination to control the power supply to apply current gradually and at stable voltage and temperature levels to the laser diode,
a temperature controller which controls the temperature of said laser diode, said temperature controller being connected between said laser diode power supply and said laser diode;
an optical unit which collimates light emitted from said laser diode parallel to a corresponding object or optically controls, separates and combines the light emitted from said laser diode; and,
a digital display connected for displaying the calculated and computed blood glucose concentration.

The present invention is based on near-infrared radiation diffuse-reflection laser spectroscopy which measures blood glucose concentration by irradiating blood vessels with harmless electromagnetic radiation. This invention uses electromagnetic radiation of a wavelength that is transmitted through the skin to the measurement part, for example, a blood vessel. Since skin is mostly composed of water (H₂O), which absorbs IR radiation in nearly the entire IR spectral range, only radiation of a certain, narrow portion of the IR spectral range called the "water transmission window" will be transmitted through the skin.

Until recently, the water transmission window was thought to only include wavelengths between 3-5 µm. However, according to investigations by the present inventors, the wavelength which is able to reach a blood vessel through the water transmission window includes wavelengths between 1.3∼1.9 µm.

Accordingly, the present invention uses electromagnetic radiation with a wavelength of 1.3µm 1.9µm radiation from a semiconductor diode laser. When electromagnetic radiation of these wavelengths irradiates the skin, light is transmitted through the skin to the blood vessel where the light interacts with the heterogeneous components of the blood. The light which reaches the blood is then diffusely relected by the blood. The reflected light will have been modulated by the characteristic vibrations of the molecules which are major components of blood.

In the present invention, the diffusely reflected light described above is integrated by an integrating sphere. The photons (hν) integrated as described above, are converted into an electrical measurement value by a detector, and that value is supplied to a processing means, such as a one-chip microcomputer. The one-chip microcomputer calculates the blood glucose concentration using an accurate calibration method. Near-infrared radiation is defined in the present invention (in accordance with the International Union of Pure and Applied Chemistry (IUPAC) definition) as follows; frequency of about 10¹³∼3.75×10¹⁴Hz; energy of about 3.984-162.57 kJ/mol (0.951 35.8Kcal/mol), 0.412∼1.55eV; wavelength of about 0.8∼30µm. The present invention is based on physical and chemical principles describing the vibrational motion of the blood glucose molecules as measured with near-infrared radiation diffuse-reflection laser spectroscopy. Such vibrational motion includes both rotational and translational motion, and includes overtone vibrations and combination vibrations. Of these vibrations, the overtone vibrations are dominant.

The analysis method incorporated in the present invention includes a mathematical model based on multiple linear regression analysis and multivariate analysis as modified by the present inventors to determine the blood glucose concentration.

The present invention provides a method and apparatus for measuring blood glucose concentration, which has the advantage of ease of use and minimal expense for patients. The present invention has no consumable parts and is portable, allowing easy out-of-home testing. The present invention is more convenient than the prior art techniques. Also, this invention does not present the possible physical damage associated with the long-term use of syringes.

The measurement apparatus of the present invention can measure blood glucose concentration in a short time and unobtrusively. Therefore, the prior art techniques, with their inconvenience and expense are rendered obsolete.

The object and other objects of the present invention are achieved by measuring blood glucose concentration in a non-invasive technique, where:
a power source, for example, a battery is supplied to a one-chip microcomputer, a digital display, a laser diode power supply, a detector (as needed), and an optical unit (as needed) by means of a power switch. The one-chip microcomputer controls the laser diode power supply so that it gradually applies current at a stable voltage and temperature to the laser diode, which emits the necessary wavelengths of radiation by means of a start/reset switch. The one-chip microcomputer is operated so that a D/A converter controlled by said one-chip microcomputer and driving said laser diode power supply converts a digital control signal into an analog control signal.

Thus, the laser diode power supply causes the laser diode to emit a wavelength suitable for measurement. The light from said laser diode is collimated, or otherwise optically controlled, separated and combined. The optically controlled light is used to irradiate the skin adjacent to a blood vessel. The light absorbed, dispersed and diffusely reflected by the blood back through the skin is integrated by an integrating unit, also termed an "integrating sphere". The photons collected by the integrating sphere are converted into an analog electrical signal with a detector. The analog electrical signal is transmitted to a preamplifier where the analog electrical signal is amplified. The amplified analog electrical signal is provided to an analog to digital (A/D) converter that converts the amplified analog electrical signal to a corresponding digital signal and outputs the digital signal to a one-chip microcomputer. The one-chip microcomputer calculates a blood glucose concentration from the digital signal by reference to a calibration curve stored in the memory of the one-chip microcomputer. The one-chip microcomputer causes the calculated blood glucose concentration to be displayed on a digital display.

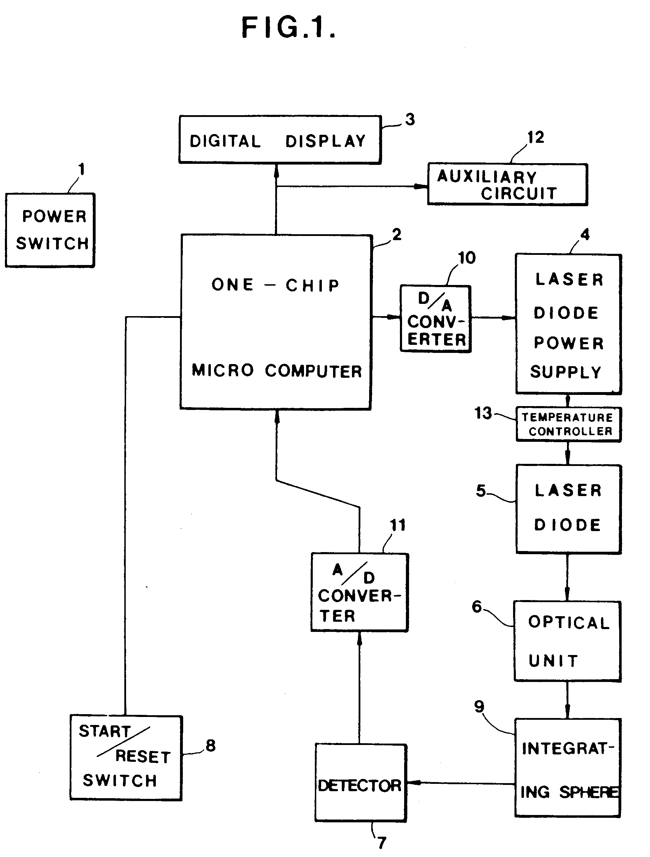

A portable apparatus for measuring blood glucose concentration using a non-invasive technique according to the present invention comprises: one-chip microcomputer which controls the laser diode power supply so that current is gradually applied to a laser diode at a stable voltage and temperature. The one-chip microcomputer calculates the blood glucose concentration by comparing a detected value with a calibration curve stored in the memory of the one-chip microcomputer. A D/A converter converts the digital control signal output from said microcomputer into an analog control signal for control of the laser diode power supply that supplies power to the laser diode. The laser diode is a light source for the blood glucose concentration measurement. There may be a plurality of laser diodes for emitting light of different wavelengths or for emitting light of like wavelengths in accordance with the current supplied from the laser diode power supply. A temperature controller (13; Figure 1) for controlling the temperature of the laser diode is connected between the laser diode power supply and the laser diode. An optical unit collimates the light emitted from the laser diode, or optically controls, separates and combines the light from the laser diode. An integrating sphere integrates the light dispersed and diffusely reflected from the blood when the blood is illuminated through the skin by light from the optical unit. A detector converts the photons collected by the integrating sphere into an analogue electrical value which is then amplified in the preamplifier. An A/D converter converts the electrical analogue measurement value into a digital value. A digital display displays the blood glucose concentration calculated by the one-chip microcomputer.

### BRIEF DESCRIPTION OF THE DRAWINGS

These and other objects and features of the present invention will be understood through the various embodiments by reference to the accompanying drawings in which:
Figure 1 is a block diagram showing an apparatus for measuring blood glucose concentration according to the present invention; and

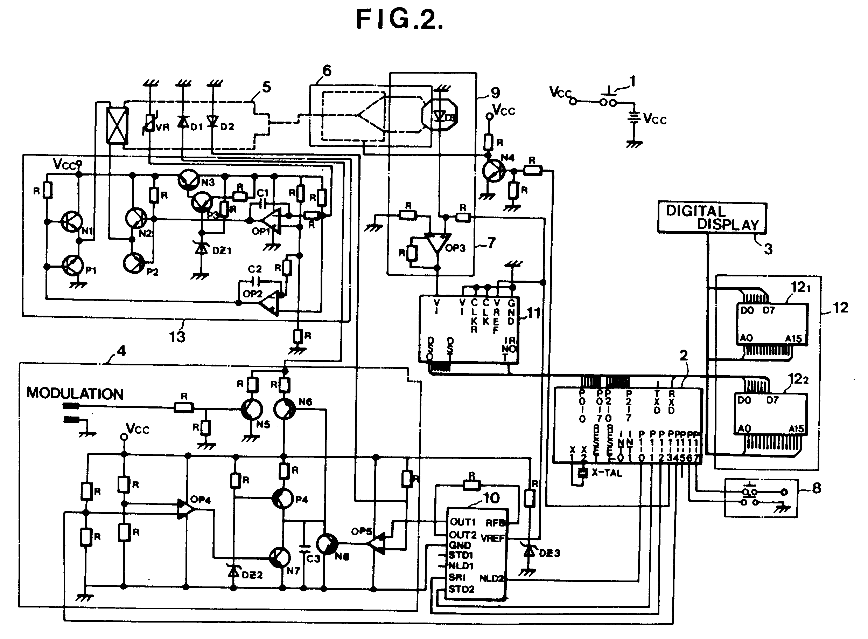

Figure 2 is a detailed circuit diagram showing the apparatus for Figure 1.

### DETAILED DESCRIPTION OF THE INVENTION

A preferred embodiment of a measurement apparatus according to the present invention will be described hereinafter with reference to the accompanying drawings.

Referring to Figure 1, when a power switch 1 is switched ON, a power is supplied from the battery (generally 4.5∼9V and is suited to a charging battery of 6V among other possibilities) to a one-chip microcomputer 2. At the same time, the power source is supplied to a digital 3, a laser diode power supply 4, and optical unit 6 (as needed).

If the start/reset switch 8 is then switched ON, the laser diode power supply 4 supplies the laser diode 5 with power in accordance with the control signal supplied by the one-chip microcomputer 2. As a result, the laser diode current gradually increases if the current exceeds the threshold current (approximately 20mA). Thus, laser diode 5 starts emitting light.

The laser diode 5 emits light (for example, light having a wavelength of 1.3µm∼1.9µm, and light having a wavelength of 1.4µm∼1.8µm, among others), of a wavelength necessary for blood glucose concentration measurement. This wavelength is achieved by gradually increasing the current supplied with in the range of approximately 20∼200mA at a stable voltage and temperature in accordance with the characteristics of the laser diode. In the present invention, the laser diode 5 is composed of between 1 and 30 diodes, and each may emit light of a different wavelength, or each may emit light of the same wavelength.

The light emitted from the collective diodes in the laser diode 5 may be simultaneously emitted by the diodes or sequentially emitted by each diode. In case of simultaneous operation, length will be selected, for example, using the Fourier Transform.

The light outputted from the collective diodes of the laser diode 5 is supplied to an optical unit 6, and collimated, or the light is optically controlled, separated and combined. Thereafter, the light is passed through an integrating sphere 9 and divided in one or more directions.

The light which passes through the integrating sphere 9 is successively irradiated to the skin of a subject, or is successively irradiated to a reference port which was ready beforehand as the case may be. Here, the reference port is not necessarily needed.

The light absorbed, dispersed and diffusely reflected from the blood is detected by a detector 7 after being integrated by means of the integrating sphere 9. The integrating sphere is of a globular or like shape. Here, the size of integrating sphere 9, which is integrated with light dispersed and reflected from the blood, has a width, length and height under 2.56cm, and is suitable for under 1.28cm, and more particularly, is suitable for under 0.64cm.

An electrical analog measurement value detected as above described is amplified by a preamplifier connected to the detector 7. Thereafter, the electrical analog measurement value is converted into a digital measurement value by means of an A/D converter 11.

Next, the one-chip microcomputer 2 calculates and computes the measured value by comparing the signal converted into a digital measurment value by the A/D converter 11 with a calibration curve stored in memory of the one-chip microcomputer 2. The resultant value is displayed on the digital display 3.

The dimensions of the above-described apparatus may be: width×length×height under 170mm×80mm×25mm, and is suitable for under 150mm×75mm×22mm, among others, and more particularly, is suitable for under 130mm×70mm×20mm.

A photo diode is suitable as the detector 7 and may be a Ge detector, and more particularly, may be a Ge detector connected to a preamplifier. Moreover, the optical unit 6 is composed of components which constitute a light which has a diameter under 0.5∼5mm (under 2mm among others) in order to condense and diffuse the light in parallel.

Furthermore, the present invention is not limited to an integrating sphere 9 having a globular or like shape, but it may also be of an oval or a half-oval or different shape.

In the present invention, the port may be separated, that is moved apart, from the above measurement apparatus while remaining connected to the apparatus by means of an optic fibre. In this case, the light emitted from the laser diode 5 can be transmitted to the port through the optic fibre, and the distance between the port and the measurement apparatus is 100∼1,000 mm and is suitable for 500 mm among others, and more particularly, may be 300 mm. Of course, the port must remain connected to the measurement apparatus.

The present invention is not limited for one-chip micro-computer 2 separated from the D/A converter 10 and the A/D converter 11, but can also be a one-chip microcomputer 2 which is included in the D/A converter 10 and the A/D converter 11.

Moreover, in the present invention, it can be used with auxiliary circuit 12 which is composed of RAM 12₁ and EPROM₂ in order to aid an operation of one-chip microcomputer 2.

The present invention is not limited for the measurement of blood glucose concentration and, for example, can be applied to a measurement of a cholesterol concentration or an alcohol concentration.

According to the present invention as above described, there is provided an economic method and apparatus for measuring blood glucose concentration in a non-invasive technique, which can easily measure the blood glucose concentration by putting the port of the apparatus to a certain part of the human body sight of a blood vessel without using an equipment, such as a conventional injector.

## Claims

1. A method of measuring the concentration of a chemical carried in a blood stream, the method comprising the steps of:
irradiating a blood vessel carrying the blood stream with near-infrared light having a wavelength of between substantially 1.3µm and substantially 1.9µm;
detecting radiation dispersed or diffusedly rejected by the blood using a photodetector (7); and,
analysing the detected radiation using diffuse-reflection spectroscopy responsive to vibrational, rotational and translational motion of the molecules of the said chemical utilising overtone vibrations and a combination of other types of vibration to determine the concentration of the chemical in the blood, the method characterised in that:
the step of irradiating is carried out with light from a laser diode (5);
the step of analysing the detected radiation is carried out by comparison of a detected value with a stored calibration curve; and,
the apparatus used to measure the concentration is a portable apparatus.

2. A method according to claim 1 in which the chemical concentration measured is blood glucose concentration.

3. A method according to claim 2 comprising the steps of:
supplying a power source from a battery to a processing means (2), a digital display (3), a laser diode power supply (4), a detector (7) and an optical unit (6) by means of a power switch (1);
controlling said processing means (2) by means of start/reset switch (8) so that said laser diode power supply (4) gradually applies current to said laser diode (5) at a stable voltage and temperature;
controlling said processing means (2) so that a D/A converter (10) coupled between said processing means (2) and said laser diode power supply (5) converts a digital control signal into an electrical control signal;
emitting a wavelength sufficient for measurement in said laser diode (5) by means of said laser diode power supply (4);
collimating the light emitted from the laser diode (5) parallel to a corresponding object, or optically controlling, separating and combining the light emitted from said laser diode (5);
irradiating through an integrating unit (9) said optically controlled light to a blood vessel to measure blood glucose concentration;
integrating by means of said integrating unit (9) the light absorbed, dispersed and diffusely reflected by the blood after the light has passed through the skin to the blood;
transmitting the signal amplified by a preamplifier connected to said detector to an A/D converter (11), after converting photons integrated by said integrating unit (9) and detected by said detector (7) into an electrical analog measurement value the step of analysing the output comprising:
transmitting the signal to said processing means (2), after converting said electrical analog measurement value into a digital measurement value by means of said A/D converter (11);
calculating and computing blood glucose concentration by comparing a calibration curve stored in memory region of said processing means (2) with a digital measurement value converted by said A/D converter (11);
displaying a calculated blood glucose concentration on said digital display (3).

4. A method for measuring blood glucose concentration according to claim 3 wherein in the step of calculating and computing blood glucose concentration by comparing a calibration curve stored in the memory of said processing means (2) with a digital measurement value converted by said A/D converter (11), utilizes a mathematical method based on a multiple linear regression analysis and a multivariate analysis.

5. An apparatus for measuring non-invasively blood glucose concentration, the apparatus comprising:
means to irradiate a blood vessel carrying the blood stream with light at a wavelength of between substantially 1.3µm and substantially 1.9µm;
an integrating unit (9) which integrates light dispersed and diffusedly reflected from the blood;
a detector (7) which amplifies a signal by means of a preamplifier after converting photons integrated by said integrating element into an electrical analog measurement value;
an A/D converter (11) for converting said electrical analog measurement value into a digital measurement value; and
processing means (2) including means for calculating and computing blood glucose concentration;
characterised in that:
the apparatus has a width x length x height less than 170 mm x 80 mm x 25 mm;
the apparatus is arranged to irradiate the blood with light from a laser diode (5);
the processing means (2) are adapted for controlling the flow of current supplied from a laser diode power supply (4) to the laser diode (5); and,
the means for calculating and computing are adapted for comparing an electrical analog measurement value detected and converted by the detector (7) with a calibration curve stored in memory of said processing means (2),
and in that the apparatus further comprises:
a D/A converter (10) for controlling the laser diode power supply by converting a digital control signal to an analog control signal, said laser diode (5) consisting of a plurality of diodes for emitting light of different wavelengths or emitting light of the same wavelength in accordance with the current supplied from said laser diode power supply, the processing means (2) and A/D converter (11) being arranged in combination to control the power supply to apply current gradually and at stable voltage and temperature levels to the laser diode (5),
a temperature controller (13) which controls the temperature of said laser diode (15), said temperature controller being connected between said laser diode power supply (4) and said laser diode (15);
an optical unit (6) which collimates light emitted from said laser diode (5) parallel to a corresponding object or optically controls, separates and combines the light emitted from said laser diode; and,
a digital display (3) connected for displaying the calculated and computed blood glucose concentration.

6. An apparatus for measuring blood glucose concentration according to claim 5, wherein the wavelength of the electromagnetic radiation emitted from said laser diode (5) is in the near-infrared region, in the range from substantially 1.4µm to substantially 1.8µm and the light emitted from said laser diode (5) simultaneously irradiates the blood through the skin, or sequentially irradiates the blood through the skin.

7. An apparatus for measuring blood glucose concentration according to claim 5 or 6, wherein said integrating unit (9) is globular or like shape, oval or half oval or other shape, and
said integrating unit has a width, length and height less than 2.56 cm, but preferably 1.28cm or less, and more particularly 0.64cm or less, and said measurement apparatus has a width x length x height less than 150mm x 75mm x 22mm, and more particularly 130mm x 70mm x 20mm or less.

8. An apparatus for measuring blood glucose concentration according to claim 5, 6 or 7 including an input/output port separable from the measurement apparatus and connected to the measurement apparatus by means of an optic fibre, and the distance between port and measurement apparatus is 100∼1,000mm and preferably 500mm or less, and more preferably is substantially 300mm.

9. An apparatus for measuring blood glucose concentration according to claims 5, 6, 7 or 8 wherein a photo diode is utilized as said detector (7) which detects photons integrated by said integrating unit (9) and a Ge detector, and preferably is coupled to a preamplifier and
said D/A converter (10) and A/D converter (11) are separated from said processing means, or are included in said processing means (2), and
said battery used as a power source is 4.5 to 9V, and is suitable for a charging battery of 6V.

## Patentansprüche

1. Verfahren zum Messen der Konzentration eines chemischen Stoffes im Blutstrom, das folgende Stufen umfaßt:
Bestrahlen eines Blutgefäßes, das den Blutstrom mit nahmen Infrarotlicht, das eine Wellenlänge zwischen etwa 1,3 µm und etwa 1,9 µm besitzt
Erfassen von Strahlung, die vom Blut in gestreuter oder diffuser Weise zurückgeworfen wird, unter Verwendung eines Photodetektors (7); und Analysieren der erfaßten Strahlung unter Verwendung von Diffusions-Reflektionsspektroskopie, ansprechend auf Vibrations-, Rotations- und Translationsbewegung der Moleküle des chemischen Stoffes, wobei Oberton-Vibrationen und eine Kombination weiterer Vibrationstypen eingesetzt werden, um die Konzentration des chemischen Stoffes im Blut festzustellen, wobei das Verfahren dadurch gekennzeichnet, daß
die Bestrahlungsstufe mit Licht aus einer Laserdiode (5) durchgeführt wird,
die Analysierungsstufe der festgestellten Strahlung durch Vergleiche eines ermittelten Wertes mit einer gespeicherten Kalibrierungskurve durchgeführt wird und
die eingesetzte Vorrichtung zum Messen der Konzentration eine tragbare Vorrichtung ist.

2. Verfahren nach Anspruch 1, dadurch gekennzeichnet, daß die gemessene chemische Konzentration eine Blutzuckerkonzentration ist.

3. Verfahren nach Anspruch 2, umfassend folgende Stufen:
Liefern einer Antriebsquelle aus einer Batterie für ein Verarbeitungsmittel (2), eine digitale Anzeige (3), eine Laserdiodenantriebsversorgung, einen Detektor (7) und eine optische Einheit (6) mittels eines Netzschalters (1);
Steuern des VerarbeitungsmittelS (2) mittels eines Start/WiederbeginnschalterS (8), so daß die Laserdiodenantriebsversorgung (4) allmählich Strom mit einer stabilen Spannung und Temperatur an die Laserdiode anlegt;
Steuern des Verarbeitungsmittels bzw. Prozessors (2), so daß ein Digital/Analog-Wandler (10), der zwischen dem Prozessor (2) und der Laserdiodenantriebsversorgung (5) eingekoppelt ist, ein digitales Steuerungssignal in ein elektrisches steuerungssignal umwandelt;
Emittieren einer Wellenlänge, die zur Messung in der Laserdiode (5) ausreichend ist, mittels der Laserdiodenantriebsversorgung (4),
Justieren des aus der Laserdiode (5) emittierten Lichts parallel zu einem entsprechenden Gegenstand, oder optisches Steuern, Trennen und Kombinieren des aus der Laserdiode (5) emittierten Lichts;
Bestrahlen eines Blutgefäßes mit dem optisch gesteuerten Licht durch eine Integrationseinheit (9), um die Blutzuckerkonzentration zu messen;
Integrieren des Lichts mittels der Integrationseinheit (9), das durch das Blut absorbiert, gestreut und diffus reflektiert wurde, nachdem das Licht durch die Haut ins Blut hindurchgetreten ist,
Übertragen des durch einen Vorverstärker verstärkten Signals, der über den Detektor mit einem Analog/Digital-Wandler (11) verbunden ist, nachdem die Photonen, die durch die Integrationseinheit (9) integriert und durch den Detektor (7) erfäßt worden sind, in einen elektrischen Analogmessungswert umgewandelt wurden, wobei die Stufe des Analysierens der Ausgangsleistung folgendes umfaßt:
Übertragen des Signals zum Prozessor (2), nachdem der elektrische Analogmessungswert in einen Digitalmessungswert mittels des Analog/Digitalwandlers (11) umgewandelt worden ist;
Errechnen und Auswerten der Blutzuckerkonzentration durch Vergleich einer Kalibrierungskurve, die im Speicherbereich des Prozessors (2) gespeichert ist, mit einem Digitalmeßwert, der durch den Analog/Digitalwandler (11) umgewandelt ist;
Anzeigen einer errechneten Blutzuckerkonzentration auf der Digitalanzeige (3).

4. Verfahren zum Messen der Blutzuckerkonzentration nach Anspruch 3, wobei in der Stufe des Errechnens und Auswertens der Blutzuckerkonzentration durch Vergleich einer Kalibrierungskurve, die im Speicher des Prozessors (2) gespeichert ist, mit einem Digitalmeßwert, der durch den Analog/Digitalwandler (11) umgewandelt worden ist, ein mathematisches Verfahren einsetzt, das auf einer multiplen linearen Regressionsanalyse und einer multivariablen Analyse basiert.

5. Vorrichtung zum nicht-inversivem Messen der Blutzuckerkonzentration, wobei die Vorrichtung folgendes umfaßt:
Mittel zum Bestrahlen eines Blutgefäßes, das den Blutstrom trägt, mit Licht bei einer Wellenlänge zwischen etwa 1,3 µm und etwa 1,9 µm;
eine Integrationseinheit (9), die das vom Blut gestreute und diffus reflektierte Licht integriert;
einen Detektor (7), der ein Signal mittels eines Vorverstärkers verstärkt, nachdem die Photonen, die durch das Integrationselement integriert worden sind, in einen elektrischen Analogmeßwert umgewandelt worden sind;
einen Analog/Digitalwandler (11) zum Umwandeln des elektrischen Analogmeßwertes in einen Digitalmeßwert, und
einen Prozessor (2), einschließlich Mittel zum Errechnen und Bewerten der Blutzuckerkonzentration,
dadurch gekennzeichnet, daß:
die Vorrichtung eine Breite x Länge x Höhe geringer als 170 mm x 80 mm x 25 mm besitzt;
die Vorrichtung so angeordnet ist, daS sie das Blut mit Licht aus einer Laserdiode (5) bestrahlt;
der Prozessor (2) ausgelegt ist, den Fluß des Stromes zu steuern, der von einer Laserdiodenantriebsversorgung (4) zur Laserdiode (5) geliefert wird; und
Mittel zum Errechnen und Bewerten ausgelegt sind, einen elektrischen Analogmeßwert, der durch den Detektor (7) erfaßt und umgewandelt worden ist, mit einer Kalibrierungskurve zu vergleichen, die im Speicher des Prozessors (2) gespeichert ist, und
daß die Vorrichtung ferner folgendes umfaßt:
einen Digital/Analogwandler (10) zum Steuern der Laserdiodenantriebsversorgung durch Umwandlung eines Digitalsteuerungssignals in ein Analogsteuersignal, wobei die Laserdiode (5) aus einer Vielzahl von Dioden besteht, die Licht unterschiedlicher Wellenlängen emittiert oder Licht der gleichen Wellenlänge in Übereinstimmung mit einem Strom zu emittieren, der von der Laserdiodenantriebsversorgung geliefert wird, und wobei der Prozessor (2) und der Analog/Digitalwandler (11) in Kombination angeordnet sind, um die Antriebskraft zu steuern, um Strom allmählich mit stabiler Spannung und Temperatur an der Laserdiode (5) anzulegen,
eine Temperatursteuerung (13), die die Temperatur der Laserdiode (15) steuert, wobei die Temperatursteuerung zwischen der Laserdiodenantriebsversorgung (4) und der Laserdiode (15) geschaltet ist,
eine optische Einheit (6), die Licht parallel zu einem entsprechenden Gegenstand justiert, das von der Laserdiode (5) emittiert wird, oder optisch steuert, trennt und das aus der Laserdiode emittierte Licht vereint, und
eine Digitalanzeige (3), die zum Anzeigen der errechneten und bewerteten Blutzuckerkonzentration angeschlossen ist.

6. Vorrichtung zum Messen der Blutzuckerkonzentration nach Anspruch 5, bei der die Wellenlänge der elektromagnetischen Strahlung, die von der Laserdiode (5) emittiert wird, im nahen Infrarotbereich liegt, im Bereich von etwa 1,4 µm bis etwa 1,8 µm, und wobei das von der Laserdiode (5) emittierte Licht gleichzeitig das Blut durch die Haut hindurch bestrahlt oder reihenfolgemäßig das Blut durch die Haut bestrahlt.

7. Vorrichtung zum Messen der Blutzuckerkonzentration nach Anspruch 5 oder 6, bei der die Integrationseinheit (9) kugelförmig oder von ähnlicher Form, oval oder halboval oder von anderer Form ist, und
daß die Integrationseinheit eine Breite, Länge und Höhe geringer als 2,56 cm, jedoch vorteilhafterweise 1,28 cm oder geringer, und in besonderster Weise 0,64 cm oder geringer aufweist, wobei die Meßvorrichtung eine Breite x Länge x Höhe geringer als 150 mm x 75 mm x 22 mm besitzt, und insbesondere 130 mm x 70 x 20 mm oder geringer.

8. Vorrichtung zum Messen der Blutzuckerkonzentration nach Anspruch 5, 6 oder 7, einschließlich einer Eingangs/Ausgangsöffnung, trennbar von der Meßvorrichtung und mit derselben mittels eines Lichtleiters an dieselbe angeschlossen ist, und wobei die Entfernung zwischen Öffnung oder Zugang und Meßapparat 100 bis 1000 mm und vorteilhafterweise 500 mm oder geringer, und noch vorteilhafterweise etwa 300 mm beträgt.

9. Vorrichtung zum Messen der Blutzuckerkonzentration nach den Ansprüchen 5, 6, 7 oder 8, wobei eine Photodiode als Detektor (7) eingesetzt wird, die die Photonen erfaßt, welche durch die Integrationseinheit (9) und einen Ge Detektor integriert werden, und die vorteilhafterweise an einen Vorverstärker angeschlossen ist und
wobei der Digital/Analogwandler (10) und der Analog/Digitalwandler (11) voneinander durch den Prozessor getrennt sind oder im Prozessor (2) aufgenommen sind und
daß die als Kraftquelle eingesetzte Batterie eine 4,5 bis 9 V Batterie ist, welche eine Batterie von 6 V laden kann.

## Revendications

1. Méthode de mesure de la concentration d'une substance chimique transportée dans un flux sanguin, la méthode comprenant les étapes consistant à :
illuminer un vaisseau sanguin transportant le flux sanguin avec une lumière dans le proche infrarouge ayant une longueur d'onde entre sensiblement 1,3 µm et sensiblement 1,9 µm ;
détecter le rayonnement dispersé ou rejeté de manière diffuse par le sang en utilisant un photodétecteur (7) ; et
analyser le rayonnement détecté en utilisant la spectroscopie à réflexion diffuse sensible au mouvement de vibration, de rotation et de translation des molécules de ladite substance chimique en utilisant les vibrations harmoniques et une combinaison d'autres types de vibrations pour déterminer la concentration de la substance chimique dans le sang, la méthode étant caractérisée en ce que :
l'étape de rayonnement est exécutée avec une lumière provenant d'une diode laser (5) ;
l'étape d'analyse du rayonnement détecté est exécutée par comparaison d'une valeur détectée avec une courbe d'étalonnage mémorisée ; et
l'appareil utilisé pour mesurer la concentration est un appareil portable.

2. Méthode selon la revendication 1, dans laquelle la concentration de substance chimique mesurée est la concentration de glucose dans le sang.

3. Méthode selon la revendication 2 comprenant les étapes consistant à :
alimenter par une source d'alimentation provenant d'une batterie des moyens de traitement (2), un affichage numérique (3), une alimentation de diode laser (4), un détecteur (7) et une unité optique (6) au moyen d'un interrupteur de puissance (1) ;
commander lesdits moyens de traitement (2) au moyen d'un commutateur de démarrage/réinitialisation (8), de sorte que ladite alimentation de diode laser (4) applique progressivement un courant à ladite diode laser (5) à une tension et à une température stables ;
commander lesdits moyens de traitement (2) de sorte qu'un convertisseur numérique-analogique (10), couplé entre lesdits moyens de traitement (2) et ladite alimentation de diode laser (5), convertisse un signal de commande numérique en un signal de commande électrique ;
émettre une longueur d'onde suffisante pour la mesure dans ladite diode laser (5) au moyen de ladite alimentation de diode laser (4) ;
collimater la lumière émise à partir de la diode laser (5) parallèle à un objet correspondant, ou commander, séparer et combiner de manière optique la lumière émise à partir de ladite diode laser (5) ;
rayonner, à travers une unité d'intégration (9), ladite lumière commandée de manière optique vers un vaisseau sanguin pour mesurer la concentration de glucose dans le sang ;
intégrer, au moyen de ladite unité d'intégration (9), la lumière absorbée, dispersée et réfléchie de manière diffuse par le sang une fois que la lumière est passée à travers la peau jusqu'au sang ;
transmettre le signal amplifié par un préamplificateur connecté audit détecteur à un convertisseur analogique-numérique (11), après avoir converti les photons intégrés par ladite unité d'intégration (9) et détectés par ledit détecteur (7) en une valeur de mesure analogique électrique, l'étape consistant à analyser la sortie comprenant :
la transmission du signal auxdits moyens de traitement (2), après avoir converti ladite valeur de mesure analogique électrique en une valeur de mesure numérique au moyen dudit convertisseur analogique-numérique (11) ;
le calcul et l'évaluation de la concentration de glucose dans le sang en comparant une courbe d'étalonnage mémorisée dans la région de mémoire desdits moyens de traitement (2) avec une valeur de mesure numérique convertie par ledit convertisseur analogique-numérique (11) ;
l'affichage d'une concentration de glucose dans le sang calculée sur ledit affichage numérique (3).

4. Méthode de mesure de la concentration de glucose dans le sang selon la revendication 3, dans laquelle l'étape consistant à calculer et à évaluer la concentration de glucose dans le sang en comparant une courbe d'étalonnage mémorisée dans la mémoire desdits moyens de traitement (2) avec une valeur de mesure numérique convertie par ledit convertisseur analogique-numérique (11), utilise une méthode mathématique basée sur une analyse à régressions linéaires multiples et sur une analyse à plus de trois dimensions.

5. Appareil de mesure de manière non invasive de la concentration de glucose dans le sang, l'appareil comprenant :
des moyens pour illuminer un vaisseau sanguin transportant le flux sanguin avec une lumière à une longueur d'onde entre sensiblement 1,3 µm et sensiblement 1,9 µm ;
une unité d'intégration (9) qui intègre la lumière dispersée et réfléchie de manière diffuse par le sang ;
un détecteur (7) qui amplifie un signal au moyen d'un préamplificateur, après avoir converti les photons intégrés par ledit élément d'intégration, en une valeur de mesure analogique électrique ;
un convertisseur analogique-numérique (11) pour convertir ladite valeur de mesure analogique électrique en une valeur de mesure numérique ; et
des moyens de traitement (2) comprenant des moyens pour calculer et évaluer la concentration de glucose dans le sang ;
caractérisé en ce que :
l'appareil a une largeur x longueur x hauteur inférieures à 170 mm x 80 mm x 25 mm ;
l'appareil est agencé de manière à illuminer le sang avec une lumière provenant d'une diode laser (5) ;
les moyens de traitement (2) sont destinés à commander le flux de courant délivré à partir d'une alimentation de diode laser (4) à la diode laser (5) ; et
les moyens pour calculer et évaluer sont destinés à comparer une valeur de mesure analogique électrique détectée et convertie par le détecteur (7) avec une courbe d'étalonnage mémorisée dans la mémoire desdits moyens de traitement (2),
et en ce que l'appareil comprend, de plus :
un convertisseur numérique-analogique (10) pour commander l'alimentation de diode laser en convertissant un signal de commande numérique en un signal de commande analogique, ladite diode laser (5) consistant en une pluralité de diodes pour émettre une lumière de longueurs d'onde différentes ou pour émettre une lumière de la même longueur d'onde en fonction du courant délivré à partir de ladite alimentation de diode laser, les moyens de traitement (2) et le convertisseur analogique-numérique (11) étant agencés en combinaison pour commander l'alimentation pour appliquer le courant progressivement et à des niveaux de tension et de température stables à la diode laser (5),
un contrôleur de température (13) qui contrôle la température de ladite diode laser (15), ledit contrôleur de température étant connecté entre ladite alimentation de diode laser (4) et ladite diode laser (15);
une unité optique (6) qui collimate la lumière émise à partir de ladite diode laser (5) parallèle à un objet correspondant, ou qui commande, sépare et combine de manière optique la lumière émise à partir de ladite diode laser ; et
un affichage numérique (3) connecté pour afficher la concentration de glucose dans le sang calculée et évaluée.

6. Appareil de mesure de la concentration de glucose dans le sang selon la revendication 5, dans lequel la longueur d'onde du rayonnement électromagnétique émis à partir de ladite diode laser (5) est dans la région du proche infrarouge, dans la plage de sensiblement 1,4 µm à sensiblement 1,8 µm, et la lumière émise à partir de ladite diode laser (5) illumine le sang à travers la peau simultanément, ou illumine le sang à travers la peau de manière séquentielle.

7. Appareil de mesure de la concentration de glucose dans le sang selon la revendication 5 ou 6, dans lequel ladite unité d'intégration (9) est de forme globulaire ou similaire, ovale ou semi-ovale ou d'une autre forme, et
ladité unité d'intégration a une largeur, une longueur et une hauteur inférieures à 2,56 cm mais, de préférence, 1,28 cm ou moins et, plus particulièrement, 0,64 cm ou moins, et ledit appareil de mesure a une largeur x une longueur x une hauteur inférieures à 150 mm x 75 mm x 22 mm et, plus particulièrement, 130 mm x 70 mm x 20 mm ou moins.

8. Appareil de mesure de la concentration de glucose dans le sang selon la revendication 5, 6 ou 7, comprenant un port d'entrée/sortie susceptible d'être séparé de l'appareil de mesure et connecté à l'appareil de mesure au moyen d'une fibre optique, et la distance entre le port et l'appareil de mesure est de 100 ∼ 1000 mm et, de préférence, de 500 mm ou moins et, plus préférablement, est sensiblement de 300 mm.

9. Appareil de mesure de la concentration de glucose dans le sang selon la revendication 5, 6, 7 ou 8, dans lequel une photodiode est utilisée comme ledit détecteur (7) qui détecte les photons intégrés par ladite unité d'intégration (9) et comme détecteur au germanium, et est, de préférence, couplée à un préamplificateur et
ledit convertisseur numérique-analogique (10) et le convertisseur analogique-numérique (11) sont séparés desdits moyens de traitement, ou sont compris dans lesdits moyens de traitement (2), et
ladite batterie utilisée comme source d'alimentation est de 4,5 à 9 V et convient comme batterie de charge de 6 V.
